(19) Europäisches Patentamt
European Patent Office
Office européen des brevets

(11) Publication number: **0 379 936**
**A2**

(12) **EUROPEAN PATENT APPLICATION**

(21) Application number: **90100834.2**

(51) Int. Cl.5: **A61K 31/44, C12Q 1/26**

(22) Date of filing: **16.01.90**

The title of the invention has been amended (Guidelines for Examination in the EPO, A-III, 7.3).

(30) Priority: **17.01.89 GB 8900924**

(43) Date of publication of application:
**01.08.90 Bulletin 90/31**

(84) Designated Contracting States:
**CH DE FR GB IT LI**

(71) Applicant: **VESTA MEDICINES (PROPRIETARY) LIMITED**
**Holpro House Snell Street**
**Micor Johannesburg(ZA)**

(72) Inventor: **Serfontein, Willem Jacob**
**Lynnwood Manor**
**Pretoria(ZA)**

(74) Representative: **Vossius & Partner**
**Siebertstrasse 4 P.O. Box 86 07 67**
**D-8000 München 86(DE)**

(54) **Pyridoxal for use in enzyme deficiencies, and a diagnostic kit for their diagnosis.**

(57) Treatment or prophylaxis of depressed or inadequate intracellular pyridoxal phosphate levels in a human or animal patient resulting from a condition, wherein the pyridoxine (PN) - pyridoxal phosphate (PLP) pathway is disturbed or insufficient, due to enzymatic insufficiencies inherent in the cells of a patient caused by genetic lack of oxidase or genetic oxidase polymorphism; cellular immaturity of premature infants; in conditions involving anemia, destruction of erythrocytes (e.g. malaria, biliary fever). The deficiencies are counteracted by the administration of pyridoxal or a precursor of pyridoxal which in vivo, once it has entered the bloodstream, is rapidly converted into pyridoxal without the intervention of oxidase or oxygen, optionally and preferably without the intervention of kinase.

EP 0 379 936 A2

# COMPOSITIONS FOR USE IN ENZYME DEFICIENCIES

The present invention relates to pharmaceutical, veterinary or dietary compositions and the new use thereof for a treatment or prophylaxis of depressed or inadequate intracellular pyridoxal phosphate levels in a human or animal patient resulting from a condition, wherein the pyridoxine (PN) - pyridoxal phosphate (PLP) pathway is disturbed or insufficient. The invention also provides new diagnostic methods and means for diagnosing such depressed or inadequate pyridoxal phosphate levels or disturbance in the pathway, more particularly for use in conjunction with the said new use.

## DISCUSSION OF PRIOR ART

The vital role of vitamin B6 (hereinafter abbreviated to B6) in health and disease has been extensively researched over the past two decades. It is now realised that many serious diseases and clinical conditions are associated with reduced blood and cellular vitamin B6 activity. However, until now many of the physiological interrelationships in animals and humans have not been known or understood. In particular there has been confusion as to whether observations relating to vitamin B6 and B6 vitamer deficiencies were results of clinical conditions or whether these deficiencies were causally related to the clinical conditions. This was due inter alia to a disregard of certain aspects of the pharmacokinetics involved, and of the role of different B6 vitamers. Prior to the present invention certain of these vitamers had never been determined systematically in biological fluids during disease processes, and indeed no suitable routine methods had existed for such systematic determinations.

Vitamin B6 occurs in three primary forms, known by their trivial names: Pyridoxine (PN), pyridoxal (PL) and pyridoxamine (PM), and their corresponding phosphorylated forms PNP, PLP and PMP. Of these, PN is the form exclusively used commercially in pharmaceutical formulations up till now. It is also the main B6 vitamer in plants.

Inside living human and animal cells, PLP is the biologically active form of vitamin B6, acting as co-enzyme in more than 100 biological reactions.

There has been a distinct prejudice in the art against the administration of B6 in any form other than that of PN. That prejudice was based not only on cost but also on the relative stability and longer shelf-life of PN. IN vitro PL is less stable. In short, there existed and still exists a severe prejudice in the art against the use of B6 vitamers other than the conventional PN such that persons skilled in the art were disinclined to accept that such other vitamers could offer any worthwhile advantages.

French patent specification 50 871 teaches compositions specifically for cardiac complaints comprising a combination of two active ingredients one being theophylline or one of certain derivatives thereof or camphor derivatives or digitalis and the other being vitamin B6. Although the specification states that vitamin B6 can be employed in the form of any of its aforesaid vitamers, the enabling disclosure, in particular that of the examples, is restricted to pyridoxine. No explanation is given of the therapeutic mechanism. Indeed the rationale behind the teachings of the reference is obscure, because it is said that the vitamin B6 as such has no effect on cardiac function. No teachings relating to the purposes and therapeutic effects contemplated by the present invention can be extracted from that French patent when read in the light of the knowledge in the art at the time. In particular the French patent does not teach the administration of vitamin B6 for the treatment or prophylaxis of actually depressed or inadequate intracellular PLP levels. Disturbances of the PN-PLP pathway are neither referred to nor suggested.

European patent application, Publication No 0270 026 in the name of the present applicant teaches a pharmaceutical composition comprising as an active ingredient pyridoxal (PL) or a physiologically compatible acid addition salt or complex of PL which in vivo rapidly releases PL. That invention teaches the use of those compounds for the treatment of or prophylaxis of vitamin B6 deficiencies caused by vitamin B6 antagonistic chemical substances formed in or released into the body by disease or by the administration of vitamin B6 antagonistic drugs. Specific situations dealt with include physiological shock, myocardial infarction, biogenic polyamines and vitamin B6 antagonistic drugs.

A recent German application (P37 05 549.6) teaches the use of pyridoxal, pyridoxamine and their phosphates for the regulation of cholesterol levels and lipid compositions in serum.

Again, no suggestion is made to apply these substances for the treatment or prophylaxis of depressed or inadequate intracellular PLP levels or to deal with disturbances of the PN-PLP pathway.

Extensive further research has now surprisingly lead to the discovery of a new class of disturbances which differs from the previously described situations in that the disturbance is due to enzymatic defects or

deficiencies inherent in the cells of the affected patient.

In the past the existence of that caurse was not known to exist. Accordingly, wherever patients failed to respond satisfactorily to conventional pyridoxine treatment, the conventional approach has been to apply increased pyridoxine doses, and sometimes very high such doses, usually applied in bolus form. Surprisingly, even such higher dosages prove ineffective in many cases and may even be toxic to the patient. Paradoxially pyridoxine then produces symptoms similar to some symptoms of a vitamin B6 shortage.

Surprisingly it has now been found that such so-called "vitamin B6 non-responsive patients" i.e. patients who do not respond adequately or at all to conventional pyridoxine therapy, do respond favourably to pyridoxal and certain precursors.

Accordingly the present invention now provides a new use of pyridoxal as an active imgredient, in an effective amount either in the form of pyridoxal (PL) itself or a precursor of pyridoxal which in vivo, once it has entered the bloodstream, is rapidly converted into pyridoxal without the intervention of oxidase or oxygen, presented as a pharmaceutical of dietary composition for the treatment or prophylaxis of depressed or inadequate intracellular pyridoxal phosphate levels in a human or animal patient resulting from a condition wherein the pyridoxine (PN) - pyridoxal phosphate (PLP) pathway is disturbed or insufficient resulting from depressed or absent enzymatic activity involving said pathway caused by an inherent cellular defect, optionally with a stabiliser or anti-oxidant and/or potentiator for the pyridoxal or precursor and pharmacologically acceptable extender, excipient or carrier substance(s) and which effective amount in the case of a pharmaceutical composition is preferably galenically formulated for infusion or for another form of sustained, continuous release of pyridoxal or said precursor to the patient, said precursor of pyridoxal being preferably one which is also rapidly converted as aforesaid without the intervention of kinase.

Such intracellular inherent defects in the enzyme activity, in particular oxydase activity occurs in cells, which are normally available for pyridoxine utilisation. Such deficiencies are of special clinical relevance under the following conditions:

(A) cellular immaturity, for example in premature infants; excessive need for replacement of damaged or destroyed cells, which replacement may occur:

(i) from less specialised precursor cells

(ii) by normal cell division

(B) genetic enzyme insufficiency, in particular oxidase deficiency or polymorphic aberration

In some cases the effects may be further aggravated by the conditions described in the said European patent application, Publication No 0270026. The present invention becomes of particular importance in situations where an increased demand for vitamin B6 arises or is expected to arise from any particular cause and has to be met by vitamin B6 supplementation. In such cases, if the patient has been identified as one of the aforesaid categories, PL supplementation rather than PN supplementation becomes essential. A particular example is the application of PL therapy or prophylaxis is when used as a means to increase the patient's B6 status before or even during major surgery or with any other critical condition, to improve the prognosis and outcome, especially in the debilitated, the elderly and those requiring massive procedures.

Since in many conditions, actual or incipient B6 deficiency (for example caused by increased PA levels arising from the condition or caused by medicaments used in the treatment) may not be suspected, the invention proposes the prophylactic inclusion of B6, partly or wholly in the form of PL, in essentially all infusion solutions and solutions used for parenteral administration. Furthermore, the inclusion of PL in infusion solutions may in general be used to decrease or control the "amine load" in the very ill patient.

No adverse effects of PL administration in experimental animals or humans are known.

The preferred compound to be used in accordance with the invention is pyridoxal itself, because that is the compound which can be utilised directly to meet the deficiency. It is the only form capable of entering directly into tissue and other B6-deprived cells, in order to be converted there directly into pyridoxal phosphate (PLP) (the only active form of B6) by the action of kinase (and its co-factor adenosine triphosphate - ATP).

PLP can be administered, according to the invention, but is less preferred, because in the gut it must first be hydrolysed to PL before absorption can occur, and also in the plasma it must first undergo hydrolysis to PL before it can enter into the cells. Although the enzymes required for such hydrolysis are usually available in the gut and in plasma, the hydrolysis is not instantaneous and delays the availability of the PL. Moreover, these enzymes can be inhibited by drugs in certain pathological conditions. On the other hand and because of these considerations, PLP can be utilised as an effective slow release form of PL, e.g. in dietary or pharmaceutical compositions for oral use in cases, where these pathological conditions do not arise.

Pyridoxamine (PM) and its phosphate (PMP) can also be used. Pyridoxamine can also enter the cell directly, in contrast to PMP, which like PLP must first by hydrolysed and in that respect suffers from the

same disadvantages. In spite of the above reasons which usually render PLP and PMP less preferred, there can be circumstances which render the inherent sustained or delayed release of PL and PM by those substances desirable.

The enzymes which are needed to convert PM into PL are normally readily available, both inside and outside of the cells, so that the reason why PL is more preferred is mainly the potential delay caused by this reaction. A possible advantage of PM is its greater stability. However, the stability of PL in pharmaceutical formulations can be enhanced satisfactorily by antioxidants such as ascorbic acid (vit C) and/or sodium metabisulphite.

Pyridoxal (as well as PM) can be employed in the form of pharmaceutically acceptable acid addition salts (e.g. the hydrochlorides) or of complexes capable of the rapid release of PL and/or PM in vivo. The following, in accordance with the invention, are examples of complexes and derivatives of pyridoxal capable of releasing pyridoxal readily in vivo, and are therefore ordinarily suitable for use instead of pyridoxal itself and in preference to pyridoxine:

I) Addition salts of PL and pharmaceutically acceptable acids, for example: HCl (preferred), $H_2SO_4$, $H_3PO_4$, certain amino acids, e.g. glutamine, asparagine, glutamic acid;

II) Acetals, resulting from the addition of an alcohol to the aldehyde group of PL. A typical example is pyridoxal monoethyl acetal hydrochloride.

III) Condensation products arising from the reaction of the aldehyde group with an amine, leading to the formation of amino alcohols or Schiff bases :

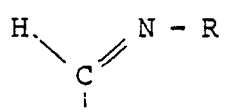

Schiff base

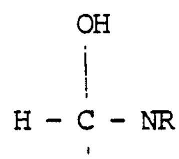

amino alcohol

The amine is preferably a biogenic or biological amine having desirable biological properties, eg.

(1) Amino acids (e.g. L-lysine, L-arginine, glycine)

(2) Pyridoxamine (PM); both PL and PLP will react with PM or PMP to form a Schiff base derivative,

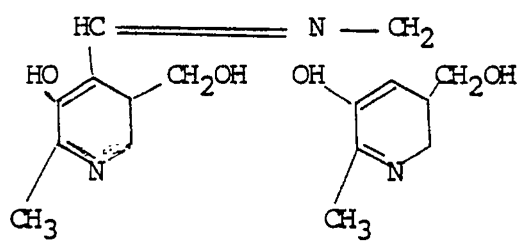

;

this Schiff base is a preferred derivative for the purposes of the present invention. In vivo it is a source of both PL and PM.

(3) Taurine

(4) Suitable diamines, e.g. ethylenediamine;

ethylenediamine reacts with PL to form a compound which in turn forms a sparingly soluble magnesium complex which may be used according to the invention as a source of both PL and Mg.

In accordance with preferred embodiments of the invention the PL or said precursor is galenically formulated for infusion or for another form of sustained, continuous release of pyridoxal or said precursor to the patient. If a precursor is used, it is preferably one which is also rapidly converted as aforesaid without the intervention of kinase. The following table is illustrative:

| Dosage rates for parenteral administration to children and adults | | | | | | | |
|---|---|---|---|---|---|---|---|
| Vitamer | Route | Daily adult dose (mg) | | | Daily dose mg/kg bodyweight | | |
| | | Range | Preferred | Optim. | Range | Preferred | Optim. |
| PL | i.v. | 2-300 | 5-150 | 10-100 | 0,03-4.28 | 0.07-2.14 | 0.14-1.43 |
| PM | i.v. | 2-300 | 5-150 | 10-100 | 0.03-4.28 | 0.07-2.14 | 0.14-1.43 |
| PM | i.v. | 2-350 | 5-250 | 10-150 | 0.03-5.0 | 0.07-3.57 | 0.14-14 |
| PLP | i.v. | 2-350 | 5-250 | 10-150 | 0.03-5.0 | 0.07-3.57 | 0.14-2.14 |
| PL | i/m | 3-400 | 5-200 | 10-150 | 0.042-5.7 | 0.07-2.89 | 0.14-2.14 |
| PM | i/m | 3-400 | 5-200 | 10-150 | 0.042-5.7 | 0.07-2.89 | 0.14-2.14 |
| PLP | i/m | 5-450 | 10-300 | 20-200 | 0.07-6.42 | 0.07-4.29 | 0.28-2.86 |
| PMP | i/m | 5-450 | 10-300 | 20-200 | 0.07-6.42 | 0.07-4.29 | 0.28-2.86 |
| PL | s/c | 3-500 | 5-250 | 10-200 | 0.042-7.14 | 0.07-3.57 | 0.14-2.86 |
| PM | s/c | 3-500 | 5-250 | 10-200 | 0.042-7.14 | 0.07-3.57 | 0.14-2.86 |
| PLP | s/c | 7-500 | 10-300 | 10-250 | 0.10-7.14 | 0.14-4.280 | 0.14-3.57 |
| PMP | s/c | 7-500 | 10-300 | 10-250 | 0.10-7.14 | 0.14-4.28 | 0.14-3.57 |
| For oral administration | | | | | | | |
| PL | | 2-500 | 5-250 | 10-200 | 0.03-7.2 | 0.07-3.6 | 0.14-2.9 |
| PM | | 2-500 | 5-250 | 10-200 | 0.03-7.2 | 0.07-3.6 | 0.14-2.9 |
| PLP | | 2-500 | 5-250 | 10-200 | 0.03-7.2 | 0.07-3.6 | 0.14-2.9 |
| PMP | | 2-500 | 5-250 | 10-200 | 0.03-7.2 | 0.07-3.6 | 0.14-2.9 |

The preferred ingredient is pyridoxal or an acid addition salt of pyridoxal.

The composition preferably has a content of pyridoxal or its said precursor in a galenic form adapted to deliver to a patient a daily dosage rate, calculated on the basis of pyridoxal of 0.03 to 4.3 mg/kg bodyweight intravenously, cr 0.04 to 5.7 mg/kg bodyweight intramuscularly, or 0.04 to 7.2 mg/kg bodyweight subcutaneously, or 0.03 to 7.2 mg/kg bodyweight orally, optionally supplied with instructions to administer it to achieve a dosage rate within the said range.

It preferably also contains a physiologically compatible antioxidant, e.g. ascorbic acid or a physiologically compatible salt of ascorbic acid or sodium metabisulphite or a mixture of any of the aforegoing with one another.

Advantageously it contains one or more potentiators for pyridoxal selected from the group consisting of magnesium ion, zinc ion and glutamate, and wherein preferably the potentiator(s) is/are present in a form and amount adapted to provide an effective dosage rate of:
in the case of magnesium ion 0.5 - 10 mg/kg/day
in the case of zinc ion 0.05 - 0.9 mg/kg/day
in the case of glutamate 0.04 - 20 mg/kg/day.

Preferred ranges are
0.1 - 0.5, more preferably 0.15 to 0.3 mg/kg/day Zn,
1.0 - 5.0, more preferably 1.5-4.3 mg/kg/day Mg, and
0.06 - 3, more preferably about 1 - 3 mg/kg/day glutamate.

The invention also contemplates a composition, which in addition contains pyridoxine or pyridoxine phosphate or a pharmaceutically acceptable complex or acid addition salt thereof in an amount not exceeding that of the pyridoxal or its precursor, and preferably also contains riboflavin as a cofactor or potentiator for pyridoxin in an amount adapted to provide an effective dosage rate of 0.05 to 0.2 mg/kg/day.

The different newly discovered situations involving inherent intracellular reduced capability or inability to utilise PN metabolically will be discussed more fully in what follows.

Cellular immaturity

Premature infants:

From a detailed comparison of the wide diversity of known facts with own findings it has now been

concluded that full term infants are usually already able to convert dietary or pharmaceutically administered PN adequately to satisfy the substantial Vitamin B6 requirements of the growing body and its metabolism, but premature infants are not or not sufficiently so able, particularly not before a stage in gestational age which in most infants is between about 28 to 30 weeks. The lower the gestational age the more severe is this deficiency. The present inventor has been able to identify, as the primary cause of this deficiency, the inability of the immature system of the infant to produce the enzyme oxidase. The deficiency is aggravated by the defective tissue-oxygenation which is often present in premature infants.

To overcome this deficiency in premature infants using conventional pyridoxine supplementation would require several tens of times more than the expected dosage rate. However, such high dosages of pyridoxine are highly undesirable, particularly when administered in bolus form. Such high dosages will then exceed the capacity of the liver, and this in turn will result in excess pyridoxine in the plasma, from where it enters the various body cells (tissue and RBC) through the cellular membranes in competition with the limited supply of PL. Once inside the cell this PN now competes against intracellular PL for whatever kinase is available in the cell to form PNP instead of the required intracellular PLP. The PNP, which is useless in the absence of adequate oxidase activity, is trapped inside the cell there to compete further with PLP for vital enzyme sites. The above described phenomena resulting from excessive dosages of pyridoxine may result in toxic effects and (paradoxically) in symptoms similar to those of vitamin B6 deficiency.

Premature infants may be treated parenterally in the acute stages followed by oral administration of PL either in the form of paediatric drops or as an ingredient of an appropriately constituted infant food formula.

Thus the invention provides a composition in the form of an infant feed formula for the treatment of premature infants suffering from intracellular enzymatic insufficiency comprising pyridoxal or said precursor in such concentration that the daily intake will be in the range 0.008 to 1.0 mg/kg bodyweight of pyridoxal, preferably 0.06 to 0.16 mg/kg.

The invention also provides a composition in the form of an additive or nutritional supplement for incorporation in an infant food regimen comprising pyridoxal or said precursor in a concentration and dosage form adapted to provide a daily intake of pyridoxal in the range of 0.008 to 1 mg/kg bodyweight, preferably in dosage units containing pyridoxal or said precursor in such concentration that the daily intake is distributed over from 1 to 6 dosage units, more particularly in the from of solid dosage units formulated for a daily intake equivalent to from 0.008 to 0.8 mg/kg bodyweight pyridoxal or pyridoxamine or from 0.013 to 1.2 mg/kg bodyweight of pyridoxal phosphate or pyridoxamine phosphate, or a mixture of the aforegoing, wherein the said intake is divided pro rata.

The invention further provides that such infant feed formula or nutritional supplement or additive as aforesaid preferably in addition contains pyridoxine or a complex or acid addition salt of pyridoxine in a concentration adapted to provide a daily intake of pyridoxin of 0.007 to 1.0 mg/kg bodyweight, and preferably in addition contains riboflavin in a concentration adapted to provide a daily intake of 0.05 to 0.2 mg/kg bodyweight.

The invention also provides a composition for administration to premature infants by infection or infusion.

Suitable dosage rates are as follows:

| For parenteral administration to premature infants | | | | | |
|---|---|---|---|---|---|
| | | Range | | Preferred Range | |
| Vitamer | Route | Dose (mg) | Dose (mg/kg) bodyweight | Dose (mg) | Dose (mg/kg) bodyweight |
| | | (daily) | (daily) | (daily) | (daily) |
| PL | i.v. | 0.03 - 3.0 | 0.01 - 1.0 | 0.01 - 0.4 | 0.03 - 0.13 |
| PM | i.v. | 0.03 - 3.0 | 0.01 - 1.0 | 0.01 - 0.4 | 0.03 - 0.13 |
| PLP | i.v. | 0.06 - 6.0 | 0.026 - 1.0 | 0.02 - 0.5 | 0.06 - 0.16 |
| PMP | i.v. | 0.06 - 6.0 | 0.024 - 2.0 | 0.02 - 0.5 | 0.06 - 0.16 |
| PL | i/m | 0.05 - 6.0 | 0.017 - 2.0 | 0.02 - 0.5 | 0.06 - 0.16 |
| PM | i/m | 0.05 - 6.0 | 0.017 - 2.0 | 0.02 - 0.5 | 0.06 - 0.16 |
| PLP | i/m | 0.06 - 9.0 | 0.02 - 3.0 | 0.03 - 0.6 | 0.1 - 0.2 |
| PMP | i/m | 0.06 - 9.0 | 0.02 - 3.0 | 0.03 - 0.6 | 0.1 - 0.2 |
| PL | s/c | 0.05 - 6.0 | 0.017 - 2.0 | 0.02 - 0.5 | 0.06 - 0.16 |
| PM | s/c | 0.05 - 6.0 | 0.017 - 2.0 | 0.02 - 0.5 | 0.06 - 0.16 |
| PLP | s/c | 0.06 - 9.0 | 0.06 - 3.0 | 0.03 - 0.6 | 0.1 - 0.6 |
| PMP | s/c | 0.06 - 9.0 | 0.06 - 3.0 | 0.03 - 0.6 | 0.1 - 0.6 |

The active B6 vitamers may be supplied in lyophilised form in dark coloured ampoules sealed under nitrogen in convenient quantities. The contents may be dissolved in saline and added to the contents of any suitable infusion solution at a rate indicated above.

For oral administration to premature infants

(i) Paediatric drops

These are formulated in an appropriately constituted flavoured base with added vitamins and minerals as in the accompanying example in such a manner that the following preferred dosage rates are achieved:

| | Range | | Preferred Range | |
|---|---|---|---|---|
| Vitamer | Dose (mg) | Dose (mg/kg) bodyweight | Dose (mg) | Dose (mg/kg) bodyweight |
| | (daily) | (daily) | (daily) | (daily) |
| PL | 0.03 - 3.0 | 0.01 - 1.0 | 0.2 - 0.5 | 0.06 - 0.16 |
| PM | 0.03 - 3.0 | 0.01 - 1.0 | 0.2 - 0.5 | 0.06 - 0.16 |
| PLP | 0.05 - 5.0 | 0.016 - 1.6 | 0.3 - 0.6 | 0.1 - 0.2 |
| PMP | 0.05 - 5.0 | 0.016 - 1.6 | 0.3 - 0.6 | 0.1 - 0.2 |

The preferred vitamer is PL.

(ii) PL-fortified infant food

An infant food is formulated complying with all the usual requirements applicable to infant food formulae with regard to protein, energy, vitamin and mineral contents. B6 vitamers are included in such a manner that the daily intake is essentially as in the previous table.

The preferred vitamer is PL itself

Alternatively tablets may be formulated for use with proprietary infant food formulae in such a manner that essentially the same amounts as listed above are administered. Such tablets are manufactured by

processes known in the art in such a way that they will dissolve and disintegrate rapidly in aqueous media such as infant food formula dilute glucose solution or in fruit juices and the quantities per tablet may be such that the full dose is contained in one tablet (to be used only once daily) or the daily dose may be subdivided over 2 to 6 tablets to be used with two feeds (e.g. morning and evening) or more often, e.g. with every feed.

In one preferred embodiment pyridoxal (or any one of the other vitamers listed) may be used in conjunction with a small quantity of pyridoxine (and riboflavine) in the form of both pediatric drops or tablets in order to stimulate liver enzyme development in the infants in such a manner that the following preferred dosage rates are achieved.

| | Range | | Preferred range | |
|---|---|---|---|---|
| Vitamer | Dose mg | Dose mg/kg bodyweight | Dose mg | Dose mg/kg bodyweight |
| | (daily) | (daily) | (daily) | (daily) |
| PL | 0.024-2.4 | 0.008-0.8 | 0.16-0.4 | 0.053-0.13 |
| PM | 0.024-2.4 | 0.008-0.8 | 0.16-0.4 | 0.053-0.13 |
| PLP | 0.04-4.0 | 0.013-1.3 | 0.24-0.5 | 0.08 - 0.17 |
| PMP | 0.04-4.0 | 0.013-1.3 | 0.24-0.5 | 0.08 - 0.17 |
| PN | 0.02-3 | 0.007-1.0 | 0.1 - 1.0 | 0.03 -0.3 |
| Riboflavin | 0.02-3 | 0.007-1.0 | 0.1 - 0.6 | 0.03 - 0.2 |

Replacement of damaged or destroyed cells

In the human or animal host the response to disease-induced or trauma-induced cellular damage is invariably growth stimulation to replace functionally incapacitated cells. Growth processes may include division of cells to produce daughter cells or it may involve cellular replacement through activation of a chain process by means of which non-differentiated precursor cells divide and ultimately develop through various stages to replace the damaged cells such as may occur after the destruction of red blood cells in disease.

In many cases, including normal healing, the regenerating cells go through a phase of rapid replication or, as in the case of red blood cell regeneration, precursor stem cells in the bone marrow proliferate to produce a series of intermediate cells culminating in an increased red cell population. The fundamental concept upon which this aspect of the present invention is based is that such rapidly regenerating cells have vitamin B6 growth requirements that differ from those of normal cells. This concept applies to individual cells, and to individual organs (e.g. the liver), and is thus analogous to the case of the premature growing infant or fetus.

In all cells, the basic requirements for growth are energy, proteins (amino acids), lipids, carbohydrates and a variety of co-factors (vitamins, minerals) which are necessary for optimal enzyme action. Amongst these, vitamin B6 activity is of special significance and is frequently a limiting factor due to the fact that B6 plays such a pivotal role in protein metabolism and energy production.

A particular application of this aspect of the invention relates to the replacement of highly differentiated cells from less differentiated precursor cells. Red blood cells (RBC) are important examples, as are other cellular elements of the blood. In all such cases the intermediate or more primitive precursor cells are better able and equipped to utilise PL rather than PN as a source of vitamin B6 activity.

A common undifferentiated primitive stem cell in the bone marrow gives rise to lymphocytes, red blood cells, granulocytes, monocytes, platelets and others in the course of which it is transformed into a variety of precursor cells. In the case of the RBC formation, this process involves the following:

Undifferentiated stem cell → Proerythroblast → Basophilic erythroblast

Polychromatic erythroblast → Acidophylic erythroblast → Reticulocyte → Erythrocyte (RBC)

Similar cascades exist in the case of the other cellular elements in the blood including platelets. A fundamental observation was the fact that a bone-marrow aspirate containing the more primitive, undifferentiated cells was found to be incapable of converting PN into PLP or PL, thus demonstrating for the first time the absence of PNP-oxidase activity in such immature cells. This has extremely important practical

significance, since the developing RBC require PLP for haeme and haemoglobin synthesis.

The normal mature RBC is perfectly capable of utilising PN as a source of B6 activity, wherefore in health, when no special demands exist for RBC formation, PN is an adequate source of B6 activity in RBC. However, in any disease process during which an increased demand for RBC production may exist, the rate of RBC production may be compromised if inadequate B6 acitivity inside the cells exists, and during such an emergency the required B6 activity cannot be supplied by supplementation with PN, since the stem cells from which RBC are formed are incapable or at best have a reduced capability of utilising PN for this purpose. The developing RBC require optimal B6 activity inside the cells for various reasons including the critical B6 requirement for haemoglobin synthesis and for energy production. In the RBC, 90% of energy is derived from the process of glycolysis, several steps of which are B6 dependent, including glucose production from glycogen (B6 dependent glycogen phosphorylase) and several transamination reactions. The process is also critically dependent on the free availability of adequate quantities of magnesium.

Having regard to the aforegoing, the invention according to a particularly important aspect thereof provides a pharmaceutical composition for the treatment of conditions involving a destruction or shortage of red blood cells and/or of haemoglobin, comprising a content of pyridoxal in a dosage form and concentration designed to deliver daily to a patient from 0,01 to 7,0 mg pyridoxal per kg body mass, the pyridoxal being optionally present in the form of a complex or soluble addition salt which in vivo readily releases pyridoxal.

The invention provides the means for a new treatment of such conditions involving a new therapeutic mechanism. It is applicable to a wide range of diseases involving a shortage of haemoglobulin-synthesising red blood cells and/or of haemoglobin.

A deficiency of haemoglobin in the blood is called anaemia, of which many types are known. They all cause pallor, fatigue and breathlessness. In severe cases the nervous system may be damaged permanently, and death may result. The direct conventional treatment of one form of anaemia usually involves the administration of iron to supplement an iron deficiency, iron being needed by normal mature erythrocytes for the synthesis of haemogoblin. However, this presupposes the availability of an adequate supply of erythrocytes capable of performing such synthesis. Many forms of disease result in a shortage of these cells.

Such diseases may involve failure of the bone marrow to produce sufficient red blood cells, causing aplastic anaemia. In the past this condition could only be relieved by transfusion, bone marrow transplantation or by marrow stimulating agents including vitamin B12.

Merrill and Henderson (Am. Rev. Nütr., 1987.7, 137056) on page 144 describe the treatment of "pyridoxine-responsive" anemias with pyridoxine. In spite of the relatively high dosages (50-200 mg PN/day) and in one case even 600 mg/day) the response rate was stated to be "optional" in fewer than half of the cases. The explanation given is not in accordance with applicant's findings. Other forms of anemia are non-responsive. The reference does not support the new approach according to the present invention. The toxic side effects of high dosages of PN are confirmed by what is stated on page 147.

Haemolytic anaemia is caused by excessive haemolysis of red blood cells, causing anaemia and jaundice. Such haemolysis may have several causes, one being the various protozoal diseases in man or animals, wherein the protozoa proliferate in and destroy the red blood cells. These include in man malaria (four different plasmodium species), bartonellosis, and in animals Rift Valley fever, corridor disease, biliary fever (Babesia canis).

Red blood cells are also destroyed by other types of micro organisms, e.g. certain viruses, such as parvo virus B19.

Some of the diseases which destroy red blood cells also destroy other cells, the regeneration of which is favourably influenced by the administration of pyridoxal or its precursors according to the invention, e.g. hepatocytes in the case of malaria.

The invention also provides compositions as aforesaid for use in the treatment or management of a condition involving microbial infection causing erythrocytes destruction, e.g. for use in the treatment or management of malaria, bartonelosis, Rift Valley fever, corridor disease or biliary fever in combination with a drug for combating the microbial infection in a common package or in a common dosage form. The invention comprise said pyridoxal or precursor thereof, in combination with said drug, as a further active ingredient, in separate dosage forms in the same package, and preferably so that said separate dosage forms are packaged side by side in pairs for combined administration for equal or different periods of treatment.

A further benefit resulting from the invention in the present context is the fact that toxins and polyamines are frequently released during disease which additionally compromise the ability of the body to utilise pyridoxine. Also, some of the drugs used in combatting such diseases have that side effect.

Pyridoxal plays an important role in overcoming the resultant B6 deficiency.

In many diseases, blood polyamine levels rise sharply due to disease-induced cellular death and disintegration. Polyamines are essential constituents inside living cells, where they are essential for macromolecular synthesis, but increased concentrations outside cells such as may occur during certain diseases are toxic due inter alia to the fact that they are known to react chemically with PLP, the resulting complexes being eliminated in the urine. We have now surprisingly found that polyamines and/or other disease-produced toxins may also inhibit the B6-activating enzyme system as well as the natural PLP-hydrolysing phosphatases and that this is one of the mechanisms by which increased toxin and polyamine levels produced in disease are toxic. This is of particular relevance to RBC haemeostasis since, in general, disease-produced polyamines are to a large extent transported inside RBC, and specifically in the case of malaria there is a marked parasite-related increase of polyamines inside RBC, whereas normally there are only trace amounts of polyamines inside RBC. RBC infected with malaria parasites therefore have a reduced capacity to utilise non-phosphorylated B6 vitamers such as PN - a further advantage of supplementation with the appropriate B6 vitamer PL or PM. In addition, accumulated intraerythrocytic polyamines (in disease) will react and thus remove PL and PLP, thereby creating an increased demand for the production of these co-factors.

Anaemia may also be caused by abnormalities of the red blood cells, as happens in macrocytic anaemia or in sickle cell anaemia, and in many of these cases patients may benefit to a varying degree from the administration of PL.

Anaemia may result from nutritional causes or from severe blood loss, due to various causes, including menstrual abnormalities, and in such cases PL is superior to PN in restoring the B6 component of the deficiency.

It has now been discovered surprisingly that pyridoxal is capable of stimulating the formation of haemoglobulin even in many cases where conventional forms of therapy are ineffective or inadequate, and specifically in some cases of so-called "pyridoxine refractory" or "pyridoxine non-responsive" anaemias.

It follows from the aforegoing that the composition and method are specifically recommended for the treatment of the aforedescribed conditions, i.e. anaemia, malaria, babesia canis infections and others with similar metabolic features.

It follows further that the invention can be applied to improving blood transfusion preparations and blood substitutes by the incorporation therein of an appropriate content of pyridoxal or one of the substances referred to which release PL in vivo or similarly by one of the other non-pyridoxine type of B6 vitamers.

In contrast to some other vitamins, the physiology and biochemistry relating to B6 differs relatively little between humans and animals. Accordingly, what has been described in the aforegoing in relation to humans is applicable also to veterinary medicine. Accordingly, the scope of the invention extends to veterinary medicine in relation to all aspects of the invention. The pharmaceutical compositions may be applied in the treatment of animal diseases and surgery substantially in a manner analogous to the treatment and prophylaxis described for humans.

The following table summarises a number of microbial diseases in man or animals involving large-scale cell destruction by intracellular or extracelllular infections:

In that table a number of microbal diseases are shown. All of these are responsible for cell death of the "affected cells". The dead cells release polyamines and in many cases toxins are also released -either by the dead cells or the microbes or both, whereby PLP is depressed and the PN-PLP pathway is compromised. Where the affected cells are blood cells, they have to be replaced in the bone marrow by the "cascade" procedure described further above. These cells play an important role in the PN-PLP pathway. Stimulated by the increased cell demand these newly formed blood cells enter the circulation in an immature state in which they cannot yet perform their PN-PLP pathway function. The replacement of the remaining cells takes place by in situ cell division, followed by cell growth of these daughter cells. This process requires the availability of adequate amounts of PLP which the young cells themselves cannot supply and which availability is compromised by cell death. The invention shortcuts the PN-PLP pathway by the direct supply of PL which can enter the cells readily for in situ conversion into intracellular PLP.

| AFFECTED CELLS | INTRACELLULAR INFECTIONS VIRUS | BACTERIA RICKETTSIA | PARASITE FUNGI | EXTRACELLULAR INFECTIONS BACTERIA |
|---|---|---|---|---|
| Red blood cells | Parvovirus B19 | | Malaria | |
| T-lymphocytes | HTLV-I;CMV HIV;HHV-6 | | | |
| B-Lymphocytes | EBV;HHV-6 | | | |
| Macrophage | | Mycobacteria Salmonella Listeria | Histoplasma Blastomyces | |
| Vascular endothelium | | Rickettsiae | | |
| Gut epithelium | Enterovirus | | Cryptosporidium Isospora | Shigella Salmonella Camphylobacter |
| Enterocyte | Rotavirus Adenovirus | | | |
| Non-keratinised squamous epithelium | Papillomavirus Herpes simplex virus | | | |
| Keratinised epithelium | Papillomavirus | | Dermatophytes | |

Cont.

| AFFECTED CELLS | INTRACELLULAR INFECTIONS VIRUS | BACTERIA RICKETTSIA | PARASITE FUNGI | EXTRACELLULAR INFECTIONS BACTERIA |
|---|---|---|---|---|
| Respiratory tract epithelium | Influenza-virus<br>Resp sinsi-tielevirus | Mycoplasma | | |
| Glial cells/Neurones | Enterovirus<br>Polyoma JC | | Toxoplasma | |
| Hepatocytes | Hepatitis A virus<br>Hepatitis B virus | | Toxoplasma | |
| | Hepatitis D virus<br>Hepatitis NANB viruses<br>Yellow fever virus | | Malaria | |

HTLV-I = Human T-cell lymphotropic/leukemia virus I
HIV = Human Immunodeficiency virus      CMV = Cytomegalovirus-6
EBV = Epstein Barr virus      HHV-6 = Human Herpesvirus-6

The above comments in the context of RBC, relating to toxins, polyamines and side effects of drugs used in the treatment apply also to many of these diseases.

In addition to the organisms in the above table, rickettsia are also to be considered in such diseases as epidemic typhus, Rocky Mountain spotted fever, Q-fever, heartwater (in cattle).

Genetic enzyme polymorphic aberration

It has now been discovered quite unexpectedly that about 20% of a normal human population have a genetic aberration (polymorphism) in their enzyme system which impairs their ability to convert pyridoxine into pyridoxal. Of course, if that condition coincides with any of the situations giving rise to vitamin B6 deficiency even in otherwise "normal" persons, such persons are especially compromised when put on conventional pyridoxine supplementation. These are the so-called "poor responders" or "non-responders" to PN therapy.

The enzyme glutamate-pyruvate-transaminase (GPT) is a PLP dependent enzyme present in RBC which can be used to assess the utilisation of PN by RBC. In the population, individuals may be divided into 3 different groups corresponding to 3 polymorphic forms of the enzyme (1-1, 25%, 1-2, 50%; 2-2, 25%.

Approximately 20% of all persons studied (belonging to all 3 groups) were found to be slow responders to oral PN, RBC GPT-activity having been measured at day 0 and day 28 before and after daily oral supplementation with PN (10 mg).

It has now been found that these slow reactors have a genetically determined PN-oxidase enzyme defect.

The presence of 20% "slow-reactors" in the population now explains for the first time why in a variety of so-called B6 responsive diseases (premenstrual tension, depression, anaemia) it has frequently been found that approximately 80% of patients respond well to the drug but that the response rate is seldom 100%.

We have now surprisingly found that such patients have reduced PN-oxidase activity and that they do respond much more satisfactorily to PL or PM administration than to PN.

The above novel observations have important clinical implications. Twenty per cent of all patients previously discussed (e.g. in infections affecting RBC B6 status or infection similarly affecting B6 status of other cells or otherwise compromised B6 status due to serious disease or drugs) will be more seriously at risk than the remaining 80%. A further aspect of the present invention provides for a suitable test to determine intracellular PN-oxidase activity and thus to identify such patients which may then receive early and pre-emptive treatment as discussed more fully below.

In applying the present invention it is furthermore proposed to combine this with appropriate quantitative or semi-quantitative diagnostic tests for PNP-oxidase activity in tissue homogenates and red blood oell haemolysates. Such tests may be used to identify patients suffering from the aforesaid genetically impaired oxidase activity, who are particularly at risk in any situation where vitamin B6 supplementation is needed and who then definitely require the administration of a source of B6 other than pyridoxine.

Such test may also be employed to identify and quantify the extent of any oxidase deficiency to which this invention relates.

A suitable simplified assay will be described by way of example which may be modified to suit particular requirements. In addition, a more accurate method based on HPLC measurement of the B6 vitamers involved in the reaction will be also described.

By the judicious application of the teachings of the present invention to suitable clinical situations it has been found possible to enhance the success of conventional forms of treatment.

For these purposes the present invention provides a diagnostic kit for diagnosing depressed or absent enzymatic activity caused by an inherent cellular defect and resulting in disturbance or insufficiency of the pyridoxine (PN) - pyridoxal phosphate (PLP) pathway and preferably for use in establishing a need in a patient for a pharmaceutical or dietary composition as aforesaid comprising:

a) a blood cell hemolizing preparation, to be used in combination with reagents for denaturing and precipitating hemoglobin, preferably forming part of the kit;

b) a set of reagents including NADH for determining intracellular oxidase or the enzyme aspartate transaminase (AS7) in the hemolysate by a reaction or series of reactions, wherein NADH is consumed as an indirect measure of the oxidase or AST activity, the NADH being measurable by optical density measurement.

One embodiment of such kit is provided for the determination of oxidase activity, comprising:

(1) a buffer preparation or buffer preparations, pH 7.0 - pH 7.8, preferably pH 7.5 - 7.6;

(2) a blood cell hemolizing preparation;

(3) a standard substrate preparation of PMP or PNP or PM or PN;

(4) a set of reagents for detecting liberated ammonia, comprising α-ketoglutarate, ADP and NADH, and, as a separate item, glutamate-dehydrogenase; and preferably auxiliary reagents for denaturing and removing hemoglobin comprising chloroform, acid and alkali.

A further embodiment of such kit is provided for determining AST activity, comprising

a) a reagent for heamolizing red blood cells to produce a hemolyzate;

b) an aspartate and α-ketoglutarate substrate;

c) a combination of reagents for determining the amount of oxaloacetate produced by reaction of aspartate aminotransferase with the substrate comprising
malic acid dehydrogemase (MDH) and NADH;

d) one or more buffer preparations pH 6.5 - 7.5, preferably 7.2.

Example 1

Quantitative assay of PNP-oxidase activity in tissue and homogenates and red blood cell haemolysates

N-(5′-phospho-4′-pyridoxyl)-N′(naphthyl)ethylenediamine (800 micromoles) was added to 3.5 ml of 0.2 M Tris buffer (pH 8.0) in screw-top test tubes, and the mixture equilibrated by shaking in a water bath at 37° C. An enzyme source (tissue homogenate red blood cell haemolysate, 0.1 - 0.5 ml) was added, and the uncapped tubes allowed to incubate with shaking for 60 min, after which 1.0 ml of 10% NaOH was added to each tube. Tubes were removed from the water bath and 5.0 ml of special grade ethyl acetate added. The tubes were closed and then shaken vigorously for 3 min. The fluorescence in the organic phase was then read (excitation 320 nm, emission 410 nm).

Enzymic activity was expressed as nanomoles of products formed/h/0.1 ml of enzyme source.

Example 2

Procedure for detecting oxidase deficiency in red blood cell (RBC) haemolysates

The principle of the procedure is as follows: A RBC haemolysate is prepared to serve as the oxidase enzyme source. The haemolysate is then allowed to react with a standardised quantity of pyridoxamine phosphate (PMP) under controlled conditions (time of reaction, pH, temperature), and during this period PMP is oxidised by the enzyme to both pyridoxal phosphate (PLP) and pyridoxal (PL). The quantity of PLP + PL formed under these conditions is a direct indication of the amount of oxidase enzyme present, and thus the amount of oxidase present in the RBC haemolysate reflects the patient's oxidase enzyme status, which is expressed in terms of nanomol PLP formed per hour per gram of haemoglobin (nmol PLP/h/g Hb).

The amount of PLP + PL may be determined either by means of fluorescence analysis (of the complexes formed by PLP + PL with semi-carbazide) or by means of HPLC methodology (which also allows for individual measurement of PLP and PL).

The fluorescence procedure may be adapted for use in automated equipment, thus allowing for the routine analysis of large numbers of samples, as detailed in the following example.

Assay of pyridoxamine phosphate oxidase (PPO)

Packed RBC haemolysate (0.125 ml) was diluted to 500 µl with potassium phosphate buffer (0.133 M, pH 8.0) 50 µl was withdrawn for haemoglobin measurement by the cyanmethaemoglobin method (Blood 1957 12: 1132).

To the remaining 450 µl, pyridoxamine-5-phosphate was added to give a final concentration of 8 µM. After 2 h incubation, the reaction was stopped with an equal volume of trichloracetic acid (10% w/v) and the precipitated protein removed by centrifugation. The protein-free extract was then used for the determination of PLP + PL either by means of automated fluorometric analysis or by means of an HPLC procedure.

In the fluorometric assay, an aliquot of each protein-free extract after incubation was transferred to a sample cup of a centrifugal analyser which was programmed to mix 1 vol. sample with 3 vols. of a buffer, pH 9.5, containing $KHCO_3$ (32.5 g/l) and $K_2CO_3$ (24.1 g/l), and this was followed by 0.2 vols. of 12% (w/v) of semicarbazide hydrochloride.

The development of the fluorescent adduct with PLP and PL was then measured after 15 min. at 37° C (peak excitation wave length 360 nm) with a broad emission filter peaking at 450 nm. The amount of PLP + PL formed was determined by using standards over the range 0.05 - 0.8 uM.

Identification of subjects genetically deficient in the enzyme pyridoxamine phosphate oxide (PPO)

Using the above analytical system, the following cut-off point is adopted which allows for the identification of genetically deficient individuals.

| Type of patient | n mol PLP + PL/g Hb/h |
|---|---|
| Normal<br>Genetically deficient | above 4.30<br>below 4.30 |

Example 3

Alternative PMP (PN) - oxidase activity measurement

Principle:

oxidase

PMP PL + $H_2O_2$ + $NH_3$

α-ketoglutarate

as substrate NADH

(standard ADP glutamate dehydrogenates

solution)

(or PNP)

NAD cofactor

L-Glutamate

The released $NH_3$ is detected by the NADH-dependent glutamate-dehydrogenase reaction. $NH_3$ is converted by α-keto glutarate with NADH (reduced form of nicotine adenine dinucleotide) as coenzyme into L-glutamate. ADP (adenosine diphosphate) is a further cofactor and GLD (glutamate dehydrogenase) is the actual enzyme for that reaction. NADH is used up and this consumption is measured. The decrease in the absorption (ΔE) of NADH corresponds to the activity of the oxidase enzyme. The combined reactions have a strong pH optimum at pH 7.5 -7.6. Added ADP is a required co-factor for the GLD reaction.

Method:

Buffer I: 0.1 M TRIS - HCl, pH 7.5 (12.1 g/l)

Buffer II: 0.5 M TRIS - HCl, pH 7.5 Preparation of haemolysates:

0.5 ml Buffer I

0.1 ml packed washed red cells

0.05 ml 10% aqueous solution of TRITON-X-100

Reagent mixture:

In 10 ml Buffer I dissolve 20 mg PMP.

Add 0.5 ml reagent mixture in reaction tube and start reaction with 0.1 ml of haemolysate in 30°C waterbath in the dark. Incubate for 0 and 90 min for each haemolysate sample. Stop reaction and denaturate hemoglobin by adding 500 ul of 1N HCl and after 5 min adding 500 µl of 1N NaOH. Add 700 µl of $CHCl_3$, shake vigorously to precipitate denatured hemoglobin and centrifuge for 10 min at 10 000 G to remove haemoglobin from the supernatant of the haemoglysate which is retained.

Photometric $NH_3$ - detection:

Dissolve

20 mg α-ketoglutarate (Na-salt) (Boehringer)

10 mg ADP (Boehringer)

6 mg NADH (Boehringer)

in 10 ml Buffer II

Pipette into cuvette:
1.4 ml of supernatant of haemolysate (see above) and add
0.6 ml of above solution in Buffer II
Measure OD at 365 nm (to determine initial NADH concentration).
Add 20 µl of glutamate-dehydrogenase in glycol (Boehringer) to start $NH_3$ consuming reaction
Measure OD again when OD has stabilised (end-point).
Perform Hb determination on haemolysate.

ΔE values (change in OD at 365 nm) obtained in this manner reflect oxidase enzyme activity. Final enzyme activity values are recorded as ΔE/g haemoglobin/h.

All reagents used must be essentially $NH_3$ free. Addition of riboflavin and/or FMN has no influence.

The following typical results were obtained in a healthy adult human population:
Erythrocyte oxidase activity:
Normal patients: 20 - 150 ΔE/g Hb/h. Patients with deficient oxidase activity: below 20 ΔE/g Hb/h.

## Example 4

### Clinical trials on dogs with B. canis infection

Babesia canis infection, better known as "biliary fever", is a severe and frequently fatal disease in dogs. It is a protozoal disease involving large-scale destruction of erythrocytes (red blood cells) and loss of haemoglobin. Double blind trials were carried out on two groups of dogs presented to the clinic for treatment of severe B. canis infections. Upon admission, all animals were recumbent with fever, jaundice, and accelerated heart and respiratory rate. All animals were given standard treatment, which consisted of post surgical drip, doxycycline, trypan blue, hepavet, electrolytes and glucose. In the experimental group, a solution of PL was added to the drip (electrolytes, glucose) given to the animals. In the control group, saline was given to replace the PL solution.

The PL solution according to the invention was made up as follows (per vial):

| | | |
|---|---|---|
| Pyridoxal hydrochloride | 243 mg | (= 200 mg PL) |
| Ascorbic acid | 10 mg | |

### Experimental treatment

Experimental animals: PL solution according to the invention was given with the drip in such a manner that each patient received 50 mg of pyridoxal over a 12 h period. Each animal received only one such course of treatment.

### Control animals

These received saline instead of PL solution under similar conditions. Control animals were selected to resemble experiental animals as closely as possible in respect of race and initial haemoglobin concentration. The results are given in the following table.

| | Hb* (g/1) | RCC (X10^12/1) | Ht (1/1) | MCV (fl) | MCHC (g/dlRC) | WCC (X10^9/1) |
|---|---|---|---|---|---|---|
| E ** | 11.0 | 0.41 | 2.1 | -6 | 3.8 | 3.0 |
| E | 12.8 | 0.49 | 4.2 | 2 | -1 | -8.4 |
| E | 6.5 | 0.47 | 3.6 | 0 | -2.5 | 2.5 |
| E | 9.5 | 0.41 | 4.2 | 3 | -1.7 | 3.1 |
| E | 7.8 | 0.23 | 2.3 | 1 | -0.5 | 1.05 |
| E | 24.8 | 0.64 | 5.4 | 1 | 2.5 | 17.9 |
| Total | 70 | 2.65 | 21.8 | 1 | -8.2 | 27.15 |
| Average*** | 11.7 | 0.44 | 3.63 | 0.17 | -0.03 | 4.52 |
| | | | | | | |
| C | 3 | 0.18 | 1.19 | 1.25 | -1.5 | -3.4 |
| C | 12 | 0.53 | 4.1 | -1.7 | 0 | -4.4 |
| C | 2 | -0.01 | 1.0 | 12 | 7 | 17.8 |
| C | -1 | -0.04 | 0.8 | 6.0 | -2.7 | -0.5 |
| C | 9 | 0.42 | 3.7 | -1.2 | -1.4 | 2.1 |
| C | 6.8 | 0.24 | 3.9 | 0.3 | 4.3 | -7 |
| Total | 31.8 | 1.31 | 12.9 | 18.3 | -2.9 | 4.6 |
| Average | 5.3 | 0.22 | 2.55 | 3.17 | -0.48 | 0.76 |

Hb*: Haemoglobin (g/1)

RCC: Red cell count ($x10^{12}/1$)

Ht: Haematocrit (1/1)

MCV: Mean corpuscular volume (fl)

MCHC: Mean corpuscular haemoglobin content (g/dlRC)

WCC: White cell count ($X10^{9}/1$)

** E: denotes experimental animals C: denotes controls, E: denotes changes per day during the period between the first two series of measurements of the parameters listed. Normally these represent measurements obtained immediately after admission (or 1 day later) and again 2 to 4 days later after treatment has been started.

*** Average changes for six animals in each group over the two periods of measurement.

Conclusions

Clinical: The clinical observation was that animals on the PL infusion responded much better to treatment than controls. These animals were significantly improved after 2 to 3 days judged on the basis of movement, water and feed consumption and alertness. Normally, as in the case of the controls, the animals only reach this stage of clinical improvement, if at all, after 5 to 6 days.

Haematological: Large, significant differences are reflected in several important parameters between experimental and control groups (haemoglobin, RCC, WCC). These are fully consistent with the clinical observations. Inter alia, there had been an amazing recovery of haemoglobin levels, substantially in excess of what could be explained in terms of normal rates of formation of new mature red blood cells, capable of synthesising haemoglobin in the normal manner.

This example illustrates the benefits of PL administration in diseases involving destruction of erythrocytes. It has far-reaching implications and applications far beyond biliary fever in dogs. The erythrocytes are formed in the bone marrow, where (in humans) they take about 120 days to mature into cells capable of synthesising haemoglobin. For this synthesis pyridoxal phosphate is needed; however, the availability of pyridoxal phosphate depends on the availability of adequate activity of certain enzymes, in particular oxidase. In the immature erythrocytes a shortage of oxidase prevails, resulting in a shortage of pyridoxal phosphate and a resultant compromised ability to synthesise haemoglobin. In many pathological conditions the enzymes responsible for the formation of pyridoxal phosphate are, moreover, inhibited. In addition, the biogenic polyamines released in some pathological conditions leading to anaemia (as in biliary fever), as well as medications conventionally employed in the treatment of these diseases, aggravate the shortage of pyridoxal needed for haemoglobin synthesis.

Further clinical trials on dogs with B-canis infection

The experiment was repeated with 13 dogs (10 inbred beagles and 3 other mongrels) randomly divided into an experimental and a control group. All animals were infected with Babesia canis by injection of blood from a splenectomised donor dog which led to the development of acute Babesiosis in approximately 3-5 days. All dogs were monitored twice daily until the haematocrit values fell to 0.15 l/l when treatment was started which in the case of all animals consisted of the following:

Electrolytes solution (number 2) for the first 24th, thereafter Post-Surgisol. Both fluids were administered by means of an intravenous jugular catheter at a rate of 80 ml/kg/24h.

Trypan blue (1%) 1 ml/kg intravenous once only. Berenil 3.5 mg/kg intramuscularly 7 days after the Trypan blue. Heparin, 25 IU/kg subcutaneously three times per day for the first 2 days.

Experimental animals, in addition, received pyridoxal in their infusions at a concentration of 50 mg/l for the entire duration of fluid therapy.

Before the start of treatment, a urine dipstick and sediment examination were performed as well as a complete clinical evaluation and faecal flotation to determine the presence of helminth ova.

Assessment: Temperature pulse and respiration were measured every 6 hours during the first 3 days and a subjective assessment of habitus (grade 1-4) and appetite (1-4) was also made at the same time.

Full haematological and biochemical assessment was performed daily.

Serum alkaline phosphatase determinations were made on days 1 and 3.

Additional trial parameters and measurements consisted of Astrup determinations (before treatment and again 24 hours later) and a ten day survival rate.

Samples for haematology were drawn in evacuated EDTA tubes and for serum chemistries into plain evacuated tubes (cephalic vein).

Plasma PLP and PL as well as RBC-PLP and PL values were determined by means of a HPLC procedure routinely used for human studies.

The following results were obtained

Two animals died in the control group and none in the experimental group. There was a definite clinical improvement in the experimental group consistent with the observations in the first experiment.

During the first 3 days (the decisive period which determines survival or not) there were significant differences in normablast count profiles: a continuous downward trend in the control animals and an inverse trend in the experimental group. Reticulocyte counts responded quicker in the experimental.

Whereas alkaline phosphatase values decreased in the control group from day 1 to day 3, there was a large increase in this parameter in the experimental group. Differences between the 2 groups were large and a definite trend discernable.

The mean corpuscular volume and the mean corpuscular hemoglobin were statistically significantly increased in the experimental animals at day 5.

Example 5: Pyridoxal infusion

Dissolve in sterile distilled water and dilute to 100 ml :

| | | |
|---|---|---|
| pyridoxal hydrochloride | 4860 mg | (= 4000 mg pyridoxal) |
| ascorbic acid | 1000 mg | |
| sodium dihydrogen phosphate | 40 g | |

Adjust pH to 6.5
Distribute 5 ml portions in amber coloured ampoules and lyophilise. Heat sterilise (10 min).
Administer as an infusion by dissolving in sterile saline and admixing the appropriate quantity with any other standard infusion or drip that the patient may be receiving (electrolytes, glucose) so that the dosage rates for parenteral administration as detailed herein will be achieved.
Alternatively, dissolve in 5 - 10 ml sterile saline and administer by intramuscular injection to achieve the dosage rate as specified herein.

Example 6: Examples of stabilised solutions of PL

a) to 100 mg of PL in 20 ml pH 7.0 Sørensen buffer (0.05 M) are added:

| | |
|---|---|
| Sodium metabisulphite : | 30 - 72 mg |
| Glucose : | 68 - 136 mg |
| Vitamin C : | 20 mg |

Filter seal in brown ampoules.
Heat sterilise at 50° C, 10 days.
Make up to 100 ml with sterile, distilled water and finally sterilise by filtration. Store in brown ampoules under nitrogen. To be used as in previous example.

Example 7: Pyridoxal-supplemented blood substitute or plasma

Pyridoxal hydrochloride (e.g. in sterile, amber coloured ampoules containing 200 mg of pyridoxal) is dissolved in 5 ml sterile saline solution and transferred to any commercially available blood substitute or transfusion solution in such a manner that, during infusion, the quantity of pyridoxal administered to the patients will be essentially as hereinbefore specified.

Example 8: Pyridoxal-fortified additive for use with conventional plasma expanders and blood transfusion

Dissolve in 100 ml of sterile water and adjust pH to 6.5:

| | |
|---|---|
| Pyridoxal.HCl | 4860 mg |
| Ascorbic acid | 1000 mg |
| Sodium dihydrogen phosphate | 40 g |

Distribute in 5 ml amber coloured ampoules and lyophilise.
Dissolve contents of ampoule in 5 ml sterile saline and add to infusion solution (blood, plasma, expander) on the following basis:
1 ampoule per 2 - 5 l.

Example 9: Use of pyridoxal in the treatment of malaria

Two groups of malaria patients are treated with pyridoxal. All patients receive standard treatment considered appropriate by the clinician in attendance consisting of anti-parasitic medication with supporting therapy to combat anaemia and possible lactate acidosis.

Clinical protocol

Group 1:

These are seriously ill patients with malaria according to the WHO criteria for severe complicated malaria. Haemoglobin values are below 10g/dl in these patients with cerebral malaria who are treated in an ICU. Generally the patients present with renal failure (creatinine 250 u mol), respiratory failure, severe electrolyte and acid-base imbalance, severe parasitaemia (more than 5% infected cells), bilirubin more than 50 μ mol/l; hypotension with bleeding abnormalities.

The patients receive normal standard treatment. In addition, the patients are given pyridoxal (200 mg per ampoule) by dissolving the contents of one ampoule in 5 ml sterile saline and adding the resulting solution to a standard electrolyte infusion solution. The rate of administration is such that the patients receive 100 mg pyridoxal/24h for the first 3 days and thereafter 50 mg/day for another 2 days.

Group 2

These are less severely ill patients or patients recovering from severe malaria. They are treated conventionally in addition with pyridoxal (or less preferably pyridoxamine) by oral administration at a dosage rate as described above, in a slow-release form. Suitable dosage forms are described in Examples 10 and 11.

Example 10: Oral slow-release tablet

A granulate is prepared having the following composition (per tablet): Pyridoxal.HCl 12.12 mg, emcompress (insoluble dicalcium phosphate filler) 125 mg, 5% gelatine sol. q.s. The granules are mixed with (per tablet) 125 mg emcompress, Natrosol (hydroxy ethyl cellulose) 125 mg, magnesium stearate 6.3 mg, and further 5% gelatine gel 9.5, mixed in a turbulo mixer and pressed into tablets.

Example 11

Example 10 is repeated, using pyridoxamine.HCl instead of pyridoxal.HCl.

Example 13: Pyridoxal-fortified additive for use in infant foods

A pyridoxal-fortified additive is formulated as follows:

| | |
|---|---|
| Pyridoxal.HCl | 10.0 mg |
| Homogenised powdered milk | 100 g |

The additive may be added to any infant feed formula (preferably of the humanised type) on the basis of 1.0 g of additive per kg bodyweight per day.

Example 14: Pyridoxal-fortified infant food formula

A basic infant food formula is composed in the usual manner containing powdered homogenised milk powder with added lactose, coconut and corn oil, and sodium citrate such that the composition is as follows: Protein 1.6%; fat 3.3%; carbohydrate 67.0%.

The following quantities of vitamin and minerals are added per 20 g of this product:

| | |
|---|---|
| Pyridoxal.HCl | 0.08 mg |
| Vitamin A | 50 IU |
| Vitamin C | 4.0 mg |
| Vitamin D2 | 50 IU |
| Vitamin E | 0.5 mg (α-tocopherol) |
| Vitamin B | 0.06 mg |
| Vitamin B2 | 0.07 mg |
| Vitamin B12 | 0.17 mg |
| Niacin | 1.0 mg |
| Ca pantothenate | 0.3 mg |
| Folic acid | 5.0 mg |
| Pyridoxine.HCl | 0.02 mg |
| Citric acid | 0.013 |
| Iron (as Fe fumarate) | 2.0 mg |
| Calcium (as calcium gluconate) | 10 mg |
| Magnesium (as Mg ascorbate) | 5 mg |
| Zinc (as Zn gluconate) | 0.2 mg |
| Iodide (as KI) | 7.0 mg |

Before use, 3.0 g are dissolved in 25 ml of water. 60 - 150 ml per kg bodyweight are given to premature infants during the first few days of life, with subsequent appropriate increases and adaptations.

| Example 15: Pyridoxal-containing paediatric syrup | |
|---|---|
| Composition per ml : | |
| Vitamin A | 50 IU |
| Vitamin B | 0.06 mg |
| Vitamin B2 | 0.07 mg |
| Pyridoxal hydrochloride | 0.08 mg |
| Pyrodoxine hydrochloride | 0.02 mg |
| Glycine | 0.1 g |
| Vitamin B12 | 0.17 mg |
| Propylene glycol | 0.1 g |
| Nicotinamide | 1.0 mg |
| Vitamin C | 4.0 mg |
| Vitamin D2 | 50 IU |
| Pantothenic acid | 0.3 mg |
| Citric acid | 0.013 mg |
| Iron (as Fe fumarate) | 2.0 mg ($Fe^{++}$) |
| Calcium (as Ca gluconate) | 60 mg |
| Magnesium (as Mg ascorbate) | 8 mg |
| Zinc (as Zinc gluconate) | 0.5 mg |
| Potassium iodide | 7.3 mg |
| Lysine hydrochloride | 1.5 mg |
| Choline chloride | 0.5 mg |
| α-Tocopherol | 0.5 mg |
| Folic acid | 5.0 mg |
| Sodium saccharin | 2 mg |
| Sodium cyclamate | 10 mg |
| Keltrol F | 2 mg |
| Excipients | 25 mg |
| 1.0 ml/kg bodyweight is administered daily. | |

Example 16

To confirm the inability of immature cells to convert pyridoxine into pyridoxal and its phosphate, an aspirate of bone marrow was tested. It was incapable of converting pyridoxine into pyridoxal, demonstrating the absence of adequate oxidase activity in premature red blood cells.

Example 17

Pediatric tablets

3 types of tablets are prepared having the following compositions:

| | Per tablet (A) | Per tablet (B) | Per tablet (c) |
|---|---|---|---|
| PL | 0.21 mg | 0.10 | 0.04 |
| PN | 0.06 mg | 0.03 | 0.012 |
| Riboflavin | 0.24 mg | 0.12 | 0.05 |
| Anhydrous lactose | q.s. (30-40 mg) | | |
| Daily dose: | | | |
| Tablets A : 1 tablet<br>Tablets B : 2 tablets<br>Tablets C : 5 tabets | | | |

The soluble tablets are added in accordance with the daily supplement regimen to a conventional feed formula.

Example 18

Diagnostic kit and method for determining long term disturbance of PN-PLP pathways - Aspartate transaminase (AST) activity.

The following diagnostic test serves to determine the existence of and the extent of a long term disturbance of the PN-PLP pathway, as may result from: genetic enzymatic defects, or long term elevated levels of vitamin B6 antagonistic drugs:

According to the invention the activity of aspartate transaminase (AST), formerly better known as glutamate oxalate transaminase (GOT), in red blood cells are determined as an indicator of such disturbance. AST requires intracellular PLP as a cofactor in order to be active. Accordingly AST activity is a measure of the intracellular PLP concentration. The AST activity is determined either in absolute terms or in relation to total AST present, i.e. after saturation of the AST with PLP, extraneously introduced (as a reagent forming part of the kit) into the hemolysate.

The reagents which are provided in kit form (the inclusion of reagents which are normally available in a laboratory being optional) include the following:

a) As a reagent for hemolizing red blood cells: 10% Triton X-100 (a surfactant), to be used with Tris buffer.

b) As reagents for the production of substrate:

b1) 0.37 M aspartic acid (4.92 g aspartic acid to appr. 70 ml unbuffered 0.1 M Tris - HCl. Add conc. NaOH to facilitate solubilisation. Adjust pH to 7.2 and volume to 100 ml.

b2) α-ketoglutarate: 0.0975 g/2.5 ml Tris-HCl (pH 7.2). Enough for 30 determinations.

c) 0.84 mM PLP solution: 11.14 mg PLP/50 ml Tris buffer (optional, if relative AST activity is to be measured, see notes above)

d) Nicotine adenine dinucleotide (reduced form: NADH) as a 0.65 mg/ml solution.

e) Malate dehydrogenase (MDH)

f) 0.1 M Tris-HCl (12.1 g/l). pH should not be adjusted before aspartic acid solution has been prepared (see (b1)).

g) Chemicals for removing hemoglobin:

standardised 1N HCl

standardised IN NaOH

chloroform Method: Add 100 µl packed red cells to 500 µl Tris buffer and 50 µl 10% Triton X 100 to prepare hemolisate.

To 100 ul of the hemolisate add 420 µl aspartic acid solution, 100 µl PLP or Tris buffer. Preincubate 10 min. at 30° C. Then add 80 µl ketoglutarate and incubate for further 30 min. During this step the AST transaminates the $-NH_2$ moiety from the aspartate to the α-ketoglutarate. In the process aspartate is converted into oxaloacetate (and α-ketoglutanate into glutamate). The amount of oxalo acetate thus produced is a measure of the amount of active AST present in the hemolysate.

At end of incubation period stop reaction as follows and at the same time remove the hemoglobin. Add 500 µl 1N HCl, mix. After 5 min. add 500 µl 1N NaOH. Add 750 µl chloroform, mix thoroughly, centrifuge 15 min. at 4000 r.p.m. This causes denaturation and precipitation of the hemoglobin, leaving a clear aqueous supernatant for spectrophotometric analysis.

Pipet 0.6 ml NADH solution into cuvettes. Add 1.4 ml aqueous supernatant. Record absorbance at 365 nm. Add 8 µl MDH, mix, record absorbence at 365 nm when baseline has stabilised. In this step the oxalo acetate formed in the first step is converted by MDH and NADH into malate, whereby an equimolecular amount of NADH is consumed. The change of absorption at 365 nm ( OD) is therefore a measure of the amount of oxaloacetate produced in the first step which in turn reflects the amount of AST present in the hemolysate.

This result is calculated in relation to the hemoglobin in the hemolysate (determined according to the method of Drabkin and Austin (J. Biol. Chem. 1937, Vol. 98, p.719).

An activity index is calculated:

$$\Delta OD \times \frac{1000}{Hb} \times 0.4448 \longrightarrow \mu\ mol\ oxalo\ acetate/gHb/h$$

$\Delta OD$ = difference of optical densities; Hb = hemoglobin

"Normal" intracellular PLP-levels yield an average activity index of 20.6 ± 6.0 (standard deviation)

Typical depressed values, e.g. after prolonged antagonistic drug use, are exemplified by figures obtained with theophyllin: average 10.4, standard deviation 3.8.

The relevant teachings of applicant's earlier application, Publication No 145229/1988, e.g. with regard to formulation of compositions may be considered as supplementary to the present disclosure. The claims and subsidiary claims herein are likewise an integral part of the present disclosure.

It will be apreciated that certain of the compositions described in the aforegoing have been specifically formulated for the new use according to the invention and are therefore novel per se regardless of the treatments for which they are intended. The same applies to the diagnostic kits. Some of the precursors of PL described in the aforegoing are considered novel per se.

## Claims

1. A new use of pyridoxal as an active imgredient, in an effective amount either in the form of pyridoxal (PL) itself or a precursor of pyridoxal which in vivo, once it has entered the bloodstream, is rapidly converted into pyridoxal without the intervention of oxidase or oxygen, presented as a pharmaceutical of dietary composition for the treatment or prophylaxis of depressed or inadequate intracellular pyridoxal phosphate levels in a human or animal patient resulting from a condition wherein the pyridoxine (PN) - pyridoxal phosphate (PLP) pathway is disturbed or insufficient resulting from depressed or absent enzymatic activity involving said pathway caused by an inherent cellular defect, optionally with a stabiliser or anti-oxidant and/or potentiator for the pyridoxal or precursor and pharmacologically acceptable extender, excipient or carrier substance(s) and which effective amount in the case of a pharmaceutical composition is preferably galenically formulated for infusion or for another form of sustained, continuous release of pyridoxal or said precursor to the patient, said precursor of pyridoxal being preferably one which is also rapidly converted as aforesaid without the intervention of kinase.

2. A use as claimed in Claim 1, characterised in that said composition has a content of pyridoxal or its said precursor in a galenic form adapted to deliver to a patient a daily dosage rate, calculated on the basis of pyridoxal of 0.03 to 4.3 mg/kg bodyweight intravenously, or 0.04 to 5.7 mg/kg bodyweight intramuscularly, or 0.04 to 7.2 mg/kg bodyweight subcutaneously, or 0.03 to 7.2 mg/kg bodyweight orally and is preferably supplied with instructions to administer it to achieve a dosage rate within the said range, the instructions being preferably in written, printed or pictorial form, more particularly included in or applied on a package containing the composition.

3. A use as claimed in Claim 1 or 2, characterised in that the active ingredient is pyridoxal or an acid addition salt of pyridoxal, preferably combined with a physiologically compatible antioxidant, preferably ascorbic acid or a physiologically compatible salt of ascorbic acid or sodium metabisulphite or a mixture of any of the aforegoing with one another.

4. A use as claimed in any one or more of Claims 1 to 3, characterised in that the composition contains

one or more potentiators for pyridoxal, more particularly selected from the group consisting of magnesium ion, zinc ion and glutamate and wherein preferably the potentiator(s) is/are present in a form and amount adapted to provide an effective dosage rate of:
in the case of magnesium ion 0.5 - 10 mg/kg/day
in the case of zinc ion 0.05 - 0.9 mg/kg/day
in the case of glutamate 0.04 - 2 mg/kg/day.

5. A use as claimed in any one or more of Claims 1 to 4, characterised in that the composition in addition contains pyridoxine or pyridoxine phosphate or a pharmaceutically acceptable complex or acid addition salt thereof in an amount not exceeding that of the pyridoxal or its precursor, and preferably also contains riboflavin as a cofactor or potentiator for pyridoxine, e.g. in an amount adapted to provide an effective dosage rate of 0.05 to 0.2 mg/kg/day.

6. A use as claimed in any one or more of Claims 1 to 5, characterised in that the composition is in the form of an infant feed formula comprising pyridoxal or said precursor in such concentration that the daily intake will be in the range 0.008 to 1.0 mg/kg bodyweight of pyridoxal, preferably in the range 0.06 to 0.16 mg/kg, or takes the form of an additive or nutritional supplement for incorporation in an infant food regimen, more particularly formulated for a daily intake as aforesaid, preferably in dosage units containing pyridoxal or said precursor in such concentration that the daily intake is distributed over from 1 to 6 dosage units, more particularly in the form of solid dosage units formulated for a daily intake equivalent to from 0.008 to 0.8 mg/kg bodyweight pyridoxal or pyridoxamine or from 0.013 to 1.2 mg/kg bodyweight of pyridoxal phosphate or pyridoxamine phosphate, or a mixture of the aforegoing, wherein the said intake is divided pro rata, and that said composition, preferably in addition contains pyridoxine or a complex or acid addition salt of pyridoxine, more particularly in a concentration adapted to provide a daily intake of pyridoxine of 0.007 to 1.0 mg/kg bodyweight, preferably in combination with riboflavin in a concentration adapted to provide a daily intake of 0.007 to 1.0 mg/kg bodyweight.

7. A use as claimed in any one or more of Claims 1 to 5, characterised in that it is for the treatment or management of a PLP deficiency in a patient suffering from a genetic lack of oxidase or genetic oxidase polymorphism.

8. A use as claimed in any one or more of Claims 1 to 5, characterized in that it is for administration to premature infants by injection or infusion.

9. A use as claimed in any one or more of Claims 1 to 5, characterised in that it is for the management or treatment of intracellular PLP deficiency in a condition involving anemia or destruction of erythrocytes, e.g. involving microbial infection causing erythrocytes desctruction, more particularly malaria, bartonelosis, Rift Valley fever, corridor disease or biliary fever.

10. A use as claimed in Claim 9, characterised in that the composition is provided in combination with a drug for combating the microbial infection in a common package or in a common dosage form, e.g. comprising said pyridoxal or precursor thereof in combination with said drug, as a further active ingredient, in separate dosage forms in the same package and preferably so that said separate dosage forms are packaged side by side in pairs for combined administration for equal or different periods of treatment.

11. A blood supplement or blood substitute or blood transfusion composition, characterised in that it contains as an active ingredient pyridoxal (PL) itself or a precursor of pyridoxal which in vivo, once it has entered the bloodstream, is rapidly converted into pyridoxal without the intervention of oxidase or oxygen and preferably formulated in the manner set out in one or more of Claims 1 to 5.

12. A use as claimed in any one or more of Claims 1 to 5, characterised in that it is in the form of an additive for addition to a blood supplement or blood substitute or blood transfusion composition.

13. A use as claimed in any one or more of Claims 1 to 10 or 12 characterised in that it is combined with means for diagnosing a condition as set out in Claim 1.

14. A diagnostic kit, for diagnosing depressed or absent enzymatic acitivity caused by an inherent cellular defect and resulting in disturbance or insufficiency of the pyridoxine (PN) - pyridoxal phosphate (PLP) pathway and preferably for use in establishing a need in a patient for a pharmaceutical or dietary composition as set out in Claim 1, comprising:

a) a blood cell hemolizing preparation, to be used in combination with reagents for denaturing and precipitating hemoglobin, preferably forming part of the kit;

b) a set of reagents including NADH for determining intracellular oxidase or the enzyme aspartate transanimose (AST) in the hemolysate by a reaction or series of reactions, wherein NADH is consumed as an indirect measure of the oxidase or AST activity, the NADH being measurable by optical density measurement.

15. A kit as claimed in Claim 14 for the determination of oxidase activity, comprising:
(1) a buffer preparation or buffer preparations, pH 7.0 - 7.8, preferably 7.5 - 7.6

(2) a blood cell hemolizing preparation;
(3) a standard substrate preparation of PM or PN; or PMP or PNP
(4) a set of reagents for detecting liberated ammonia, comprising $\alpha$-ketoglutarate, ADP and NADH, and, as a separate item, glutamate-dehydrogenase;
and optionally auxilliary reagents for denaturing and removing hemoglobin comprising chloroform, acid and alkali.

16. A kit as claimed in Claim 14 for determining AST activity, comprising
a) a reagent for heamolizing red blood cells to produce a hemolyzate;
b) an aspartate and $\alpha$-ketoglutorate substrate;
c) a combination of reagents for determining the amount of oxaloacetate produced by reaction of aspartate aminotransferase with the substrate comprising malic acid dehydrogemase (MDH) and NADH;
d) one or more buffer preparations pH 6.5 - 7.5, preferably 7.2.